# FASCICULE DE BREVET EUROPEEN

(11) **EP 0 578 591 B1**
(45) Date de publication et mention de la délivrance du brevet: **15.10.1997**
(21) Numéro de dépôt: 93420288.8
(22) Date de dépôt: 01.07.1993
(51) Int. Cl.: C07C 55/14, C07C 51/14

(54) **Procédé de préparation de l'acide adipique par hydrocarboxylation d'acides penténoiques**
Verfahren zur Herstellung von Adipinsäure durch Hydrocarboxylierung der Pentensäuren
Process for preparing adipic acid by hydrocarboxylation of pentenic acids

(30) Priorité: 09.07.1992 FR 9208762
(43) Date de publication de la demande: 12.01.1994
(73) Titulaire: RHONE-POULENC FIBER & RESIN INTERMEDIATES, 92405 Courbevoie (FR)
(72) Inventeur: Denis, Philippe, F-69150 Decines (FR); Metz, François, F-69390 Vernaison (FR); Perron, Robert, F-69390 Charly (FR)
(74) Mandataire: Vignally, Noel

(56) Documents cités:
- FR-A- 2 015 735
- US-A- 3 845 121

## Description

La présente invention concerne un procédé de préparation de l'acide adipique par hydrocarboxylation d'acides penténoïques, c'est-à-dire par réaction de l'eau et de l'oxyde de carbone sur au moins un acide penténoïque, en présence d'un catalyseur à base de rhodium et d'au moins un promoteur iodé

Dans la demande de brevet européen n° 188 209 il est proposé un procédé de préparation d'acides dicarboxyliques linéaires, en particulier de l'acide adipique, par réaction d'acides monocarboxyliques insaturés, en particulier l'acide pentène-3 oïque, de l'oxyde de carbone et d'eau, en présence d'un catalyseur à base de rhodium et d'un promoteur iodé, la réaction étant conduite dans un solvant tel le chlorure de méthylène à une température de 100 à 240°C et sous une pression totale comprise entre 14 et 240 atm; une température comprise entre 150 et 180°C et une pression totale comprise entre 24 et 40 atmosphères sont estimées préférables. La pression partielle du monoxyde de carbone est habituellement comprise entre 10 et 35 atmosphères et de préférence entre 10 et 17 atmosphéres. Le choix du solvant est indiqué être critique dans le cadre du procédé en cause et il est même estimé que des solvants tels l'acide acétique sont indésirables, en raison des faibles taux de linéarité obtenus en leur présence.

De même, il est affirmé que des solvants non polaire tels le cyclohexane et le toluéne sont eux aussi indésirables, en raison de leur propension à promouvoir directement la formation de produits branchés et indirectement celle des acides monocarboxyliques saturés.

Dans la demande de brevet européen n° 0 274 076, il est proposé un procédé de préparation d'acides carboxyliques linéaires par hydroxycarboxylation d'esters insaturés ou d'alcènes à insaturation terminale comportant de 4 à 16 atomes de carbone, en présence d'un catalyseur à base de rhodium et d'un promoteur iodé. La réaction est conduite dans un solvant choisi indifféremment parmi le chlorure de méthylène, le dichloro-1,2 éthane et les solvants aromatiques et un acide aliphatique ou aromatique de pKa compris entre 4,2 et 5,2 est présent comme accélérateur de la réaction. La pression partielle de l'oxyde de carbone est comprise entre 10 et 200 atmosphère et de préférence entre 13 et 20 atmosphères.

Toutefois au départ d'esters penténoïques on observe essentiellement la formation de monoadipate de méthyle.

Le brevet EP-A-0 477 112 décrit la préparation d'acide adipique par hydrocarboxylation d'acides penténoïques en présence de rhodium et d'un promoteur iodé, dans un solvant choisi parmi les acides carboxyliques.

Tous ces procédés de l'art antérieur sont mis en oeuvre dans un solvant d'un type ou d'un autre.

Il a maintenant été trouvé qu'il est possible d'obtenir sélectivement l'acide adipique par hydrocarboxylation d'acides penténoïques en présence d'un catalyseur à base de rhodium et d'un promoteur iodé sans utiliser de tiers solvant, c'est-à-dire en mettant en oeuvre uniquement un ou plusieurs acides penténoïques.

La présente invention a donc pour objet un procédé de préparation de l'acide adipique par réaction de l'eau et de l'oxyde de carbone sur au moins un acide penténoïque, en présence d'un catalyseur à base de rhodium et d'au moins un promoteur iodé, à une température comprise entre 100 et 240°C, sous une pression supérieure à la pression atmosphérique, caractérisé en ce que
a) la réaction est mise en oeuvre en l'absence de tiers solvant
b) et la pression partielle de l'oxyde de carbone mesurée à 25°C est inférieure ou égale à 20 bar.

Par acide penténoïque, on entend dans le cadre de la présente invention l'acide pentène-2 oïque, l'acide pentène-3 oïque, l'acide pentène-4 oïque et leurs mélanges.

L'acide pentène-4 oïque conduit à de bons résultats, mais reste peu disponible.

L'acide pentène-3 oïque pris isolément ou en mélange avec ses isomères est plus particulièrement approprié, compte-tenu de son accessibilité et des résultats satisfaisants auxquels il conduit dans le cadre du présent procédé.

Les acides penténoïques mis en oeuvre peuvent bien entendu contenir de manière minoritaire des sous-produits formés lors de leur préparation, tels que par exemple l'acide valérique, les méthyl-butyro-lactones, l'acide adipique, l'acide méthyl-glutarique et l'acide éthyl-succinique.

Le procédé selon la présente invention nécessite la présence d'un catalyseur à base de rhodium. N'importe quelle source de rhodium est susceptible d'être mise en oeuvre.

A titre d'exemples de sources de rhodium susceptibles de convenir à la mise en oeuvre du présent procédé on peut citer:
Rh métal; Rh₂O₃ ;
RhCl₃ ; RhCl₃, 3H₂O ;
RhBr₃ ; RhBr₃, 3H₂O ;
Rhl₃ ; Rh(NO₃)₃ ; Rh(NO₃)₃, 2H₂O ;
Rh₂(CO)₄Cl₂ ; Rh₂(CO)₄Br₂ ; Rh₂(CO)₄l₂ ;
Rh(CO)Cl[P(C₆H₅)₃]₂ ;
Rh[P(C₆H₅)₃]₂(CO)l ;
Rh[P(C₆H₅)₃]₃Br ;
Rh₄(CO)₁₂ ; Rh₆(CO)₁₆ ; Rh(CO)₂ (acac) ;
Rh(Cod)(acac)₂; Rh(acac)₃ ;
Rh₂(Cod)₂Cl₂ ; Rh₂(CO₂CH₃)₄ ;
HRh(CO)[P(C₆H₅)₃]₃ ;
(Cod = cyclooctadiène-1,5; acac: acétylacétonate).

Conviennent plus particulièrement à la mise en oeuvre du présent procédé:
HRh(CO)[P(C₆H₅)₃]₃ ;
Rh(CO)Cl[P(C₆H₅)₃]₂ ;
Rh₂(Cod)₂Cl₂ ;
Rh₂(CO)₄Cl₂ ;
Rhl₃ ; RhCl₃, 3H₂O ; Rh(acac)₃ ;
Rh(Cod)(acac)₂ ; Rh₂(CO₂CH₃)₄ ; Rh₄(CO)₁₂ ;
   et Rh₆(CO)₁₆.

La quantité de rhodium à mettre en oeuvre peut varier dans de larges limites.

En général, une quantité exprimée en mole de rhodium métallique par litre de milieu réactionnel comprise entre 10⁻⁴ et 10⁻¹ conduit à des résultats satisfaisants. Des quantités inférieures peuvent être mise en oeuvre ; on observe toutefois que la vitesse de réaction est faible. Des quantités supérieures n'ont d'inconvénients qu'au plan économique.

De préférence, la concentration de rhodium est comprise entre 5.10⁻⁴ et 10⁻² (inclus) mol/l.

Par promoteur iodé on entend dans le cadre du présent procédé Hl et les composés organoiodés capables de générer Hl dans les conditions réactionnelles et, en particulier les iodures d'alkyles en C₁-C₁₀, l'iodure de méthyle étant plus particulièrement préconisé.

La quantité de promoteur iodé à mettre en oeuvre est en général telle que le rapport molaire I/Rh soit supérieur ou égal à 0,1. Il n'est pas souhaitable que ce rapport excède 20. De préférence, le rapport molaire I/Rh est compris entre 1 et 4 (inclus).

La présence d'eau est indispensable à la conduite du procédé selon la présente invention. En général, la quantité d'eau à mettre en oeuvre est telle que le rapport molaire eau/acide(s) penténique(s) soit compris entre 0,01 et 10 (inclus).

Une quantité inférieure présente l'inconvénient de trop limiter la conversion. Une quantité supérieure n'est pas souhaitable en raison de la perte d'activité catalytique observée.

Selon une caractéristique essentielle du présent procédé, la pression partielle de l'oxyde de carbone, mesurée à 25°C, est inférieure ou égale à 20 bar.

Lorsque la pression partielle de l'oxyde de carbone, mesurée à 25°C, est supérieure à cette valeur la sélectivité en diacides linéaire et/ou branchés est très faible et on note la formation de quantités importantes de méthyl-4-butyrolactone, composé indésirable.

Un minimum de pression partielle de l'oxyde de carbone de 0,5 bar (mesurée à 25°C) est préconisé.

De préférence, la pression partielle de l'oxyde de carbone, mesurée à 25°C, est inférieure ou égale à 10 bar.

On peut utiliser de l'oxyde de carbone sensiblement pur ou de qualité technique, tel qu'il se présente dans le commerce.

Comme indiqué précédemment, la température de réaction est comprise entre 100 et 240°C. Pour une bonne mise en oeuvre de la présente invention, la température est comprise entre 160 et 190°C (inclus).

La réaction est conduite sous pression supérieure à la pression atmosphérique et, généralement, en phase liquide.

La pression totale peut varier dans certaines limites qui dépendront du mode opératoire retenu, de la pression partielle de l'oxyde de carbone et de celles des constituants du milieu réactionnel à la température réactionnelle choisie et, le cas échéant de la pression autogène du (ou des) acide(s) penténoïque(s) présents.

Le milieu réactionnel renferme de l'eau, une ou des sources de rhodium, un ou des promoteurs iodés et, le cas échéant tout ou partie du ou des acides penténoïques engagés et des produits de la réaction.

En fin de réaction ou du temps imparti à celle-ci, on sépare l'acide adipique par tout moyen approprié, par exemple par cristallisation.

Les exemples ci-après illustrent la présente invention.

### EXEMPLE 1

Dans une ampoule de verre préalablement purgée à l'argon, on introduit :
- 1,31 mmol de rhodium sous forme de [RhCl(Cod)]₂
- 0,35 g (2,5 mmol) de CH₃l
- 2,29 g (127 mmol) d'eau
- 55,2 g (552 mmol) d'acide pentène-3-oïque.

L'ampoule est placée dans un autoclave de 125 ml.

L'autoclave est fermé hermétiquement, placé dans un four agité et raccordé à l'alimentation de gaz sous pression. On admet à froid 2 bar de CO et on chauffe à 175°C en 20 minutes. Lorsque cette température est atteinte, on régule la pression à 8 bar.

Après une durée de réaction de 90 minutes, l'absorption de CO a cessé; l'autoclave est alors refroidi et dégazé.

La solution réactionnelle est analysée par chromatographie en phase gazeuse et par chromatographie liquide haute pression.

Les quantités de produits formés (rendement molaire par rapport à l'acide pentène-3 oïque transformé) sont les suivantes :

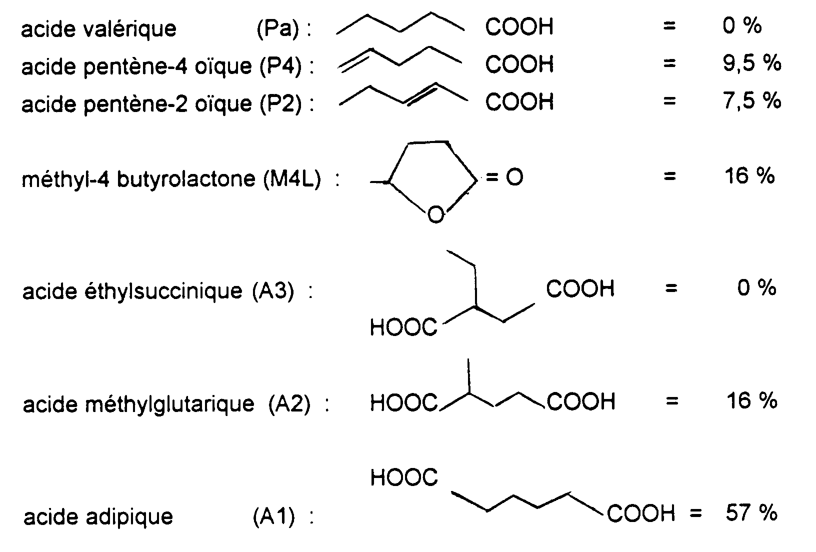

Le taux de linéarité (L) est de 77 %.

Le taux de transformation de l'acide pentène-3 oïque (TT) est de 47 %.

La productivité exprimée en gramme d'acide adipique produit par heure et par litre de mélange réactionnel (calculée sur 30 min d'absorption de CO) est de 1100 g/h.l.

### ESSAI COMPARATIF 1

On répète l'exemple 1 dans les mêmes conditions, mais avec les charges suivantes :
- 1,31 mmol de rhodium sous forme de [RhCl(Cod)]₂
- 0,35 g (2,5 mmol) de CH₃l
- 2,29 g (127 mmol) d'eau
- 4,88 g (48,8 mmol) d'acide pentène-3-oïque
- 43,2 g d'acide acétique.

On obtient les résultats suivants:
- TT de P3 : 100 %
- taux de linéarité : 73 %
- rendement en acide adipique : 62 %
- productivité : 130 g/h.l

### EXEMPLE 2

On répète l'exemple 1 en suivant son mode opératoire, dans les conditions suivantes :
- température : 175°C
- pression CO: 20 bar
- durée en température : 180 min
   et les charges suivantes :
- 0,192 mmol de rhodium sous forme de [RhCl(Cod)]₂
- 0,105 g (0,468 mmol) de Hl sous forme de solution à 57 % dans l'eau
- 2,31 g (128 mmol) d'eau
- 53,63 g (536 mmol) d'acide pentène-3-oïque

On obtient les résultats suivants :
- TT de P3 : 41 %
- taux de linéarité : 55 %
- rendement en acide adipique : 42 %
- productivité : 170 g/h.l

### EXEMPLE 3

On répète l'exemple 1 en suivant son mode opératoire, dans les conditions suivantes:
- température : 175°C
- pression CO : 8 bar
- durée en température : 205 min
   et les charges suivantes :
- 0,192 mmol) de rhodium sous forme de [RhCl(Cod)]₂
- 0,105 g (0,468 mmol) de Hl sous forme de solution à 57 % dans l'eau
- 2,31 g (128 mmol) d'eau
- 53,76 g (538 mmol) d'acide pentène-3-oïque

On obtient les résultats suivants :
- TT de P3 : 38%
- taux de linéarité : 63 %
- rendement en acide adipique : 49 %
- productivité : 150 g/h.l

## Revendications

1. Procédé de préparation de l'acide adipique par réaction de l'eau et de l'oxyde de carbone sur au moins un acide penténoïque, en présence d'un catalyseur à base de rhodium et d'au moins un promoteur iodé, à une température comprise entre 100 et 240°C, sous une pression supérieure à la pression atmosphérique caractérisé en ce que
a) la réaction est mise en oeuvre en l'absence de tiers solvant
b) et la pression partielle de l'oxyde de carbone mesurée à 25°C est inférieure ou égale à 20 bar.

2. Procédé selon la revendication 1, caractérisé en ce que la concentration en rhodium dans le milieu réactionnel est comprise entre 10⁻⁴ et 10⁻¹ mol/l (inclus).

3. Procédé selon l'une des revendications 1 ou 2, caractérisé en ce que le rapport molaire I/Rh est supérieur ou égal à 0,1.

4. Procédé selon l'une des revendications 1 à 3, caractérisé en ce que le rapport molaire I/Rh est inférieur ou égal à 20.

5. Procédé selon l'une des revendications 1 à 4, caractérisé en ce que le rapport molaire eau/acide(s) penténoïque(s) est compris entre 0,01 et 10 (inclus).

6. Procédé selon l'une des revendications 1 à 5, caractérisé en ce que la température de réaction est comprise entre 160 et 190°C.

7. Procédé selon l'une des revendications 1 à 6, caractérisé en ce que la pression partielle de l'oxyde de carbone, mesurée à 25°C, est inférieure ou égale à 10 bar.

8. Procédé selon l'une des revendications 1 à 7, caractérisé en ce que l'acide penténoïque mis en oeuvre est l'acide pentène-3 oïque seul ou en mélange avec l'acide pentène-4 oïque et/ou l'acide pentène-2 oïque.

## Claims

1. Process for preparing adipic acid by reaction of water and carbon monoxide with at least one pentenoic acid, in the presence of a rhodium-based catalyst and of at least one iodinated promoter, at a temperature between 100 and 240°C, at a pressure greater than atmospheric pressure, characterised in that
a) the reaction is implemented in the absence of a third solvent
b) and the carbon monoxide partial pressure, measured at 25°C, is less than or equal to 20 bar.

2. Process according to claim 1, characterised in that the rhodium concentration in the reaction mixture is between 10⁻⁴ and 10⁻¹ mol/l (inclusive).

3. Process according to either of claims 1 or 2, characterised in that the I/Rh molar ratio is greater than or equal to 0.1.

4. Process according to one of claims 1 to 3, characterised in that the I/Rh molar ratio is less than or equal to 20.

5. Process according to one of claims 1 to 4, characterised in that the water/pentenoic acid(s) molar ratio is between 0.01 and 10 (inclusive).

6. Process according to one of claims 1 to 5, characterised in that the reaction temperature is between 160 and 190°C.

7. Process according to one of claims 1 to 6, characterised in that the carbon monoxide partial pressure, measured at 25°C, is less than or equal to 10 bar.

8. Process according to one of claims 1 to 7, characterised in that the pentenoic acid used is penten-3-oic acid alone or as a mixture with penten-4-oic acid and/or penten-2-oic acid.

## Patentansprüche

1. Verfahren zur Herstellung von Adipinsäure durch Reaktion von Wasser und Kohlenoxid mit mindestens einer Pentensäure in Anwesenheit eines Katalysators auf der Basis von Rhodium und mindestens eines iodierten Promotors, bei einer Temperatur zwischen 100 °C und 240 °C und unter einem Druck von höher als dem atmosphärischen Druck, dadurch gekennzeichnet, daß
a) die Reaktion in Abwesenheit eines dritten Lösungsmittels durchgeführt wird
b) und der Partialdruck des Kohlenoxids, gemessen bei 25 °C, unter oder gleich 20 bar beträgt.

2. Verfahren nach Anspruch 1, dadurch gekennzeichnet, daß die Konzentration an Rhodium in dem Reaktionsmedium zwischen 10⁻⁴ und 10⁻¹ Mol/l (eingeschlossen) beträgt.

3. Verfahren nach einem der Ansprüche 1 oder 2, dadurch gekennzeichnet, daß das molare Verhältnis I/Rh höher oder gleich 0,1 beträgt.

4. Verfahren nach einem der Ansprüche 1 bis 3, dadurch gekennzeichnet, daß das molare Verhältnis I/Rh niedriger oder gleich 20 beträgt.

5. Verfahren nach einem der Ansprüche 1 bis 4, dadurch gekennzeichnet, daß das molare Verhältnis Wasser/Pentensäure(n) zwischen 0,01 und 10 (eingeschlossen) beträgt.

6. Verfahren nach einem der Ansprüche 1 bis 5, dadurch gekennzeichnet, daß die Reaktionstemperatur zwischen 160 °C und 190 °C beträgt.

7. Verfahren nach einem der Ansprüche 1 bis 6, dadurch gekennzeichnet, daß der Partialdruck des Kohlenoxids, gemessen bei 25 °C, unter oder gleich 10 bar beträgt.

8. Verfahren nach einem der Ansprüche 1 bis 7, dadurch gekennzeichnet, daß die eingesetzte Pentensäure nur 3-Pentensäure allein ist oder in Mischung mit 4-Pentensäure und/oder 2-Pentensäure vorliegt.
